# FASCICULE DE BREVET EUROPEEN

(11) **EP 0 408 455 B1**
(45) Date de publication et mention de la délivrance du brevet: **12.05.1993**
(21) Numéro de dépôt: 90402008.8
(22) Date de dépôt: 12.07.1990
(51) Int. Cl.: A61K 6/00, A61K 6/087

(54) **Composition à base de Ca(OH)2 pour la désinfection des canaux dentaires**
Ca(OH)2-Zusammensetzung zur Desinfektion von Dentalkanälen
Ca(OH)2 composition for the disinfection of dental canals

(30) Priorité: 12.07.1989 FR 8909441
(43) Date de publication de la demande: 16.01.1991
(73) Titulaire: Produits Dentaires PIERRE ROLLAND, 33708 Merignac (FR)
(72) Inventeur: Breillat, Jacques, F-75007 Paris (FR); Cohen, Anna, F-75116 Paris (FR); Langlois, Christian, F-91370 Verrières-le-Buisson (FR)
(74) Mandataire: Bruder, Michel

(56) Documents cités:
- EP-A- 0 023 994
- GB-A- 2 173 184
- GB-A- 2 173 207

## Description

La présente invention concerne une pâte de désinfection des canaux dentaires à base d'hydroxyde de calcium.

Lors des traitements destinés à conserver les dents dont on a dû éliminer la pulpe, ou dont la pulpe est nécrosée, il importe que tout le contenu organique et le maximum de microorganismes soient éliminés de l'intérieur de la dent par un nettoyage mécanique et chimique avant l'obturation définitive, pour faire disparaître ou éviter la survenue de lésions périapicales. Cette opération nécessite, en général, plusieurs séances et il est souhaitable entre chacune d'elles, d'introduire, afin de maintenir l'assainissement obtenu, un produit antiseptique dans l'endodonte, qui sera par ailleurs protégé du milieu buccal septique par un ciment de scellement provisoire.

A. Byström et Coll. (Endod. Dent. Traumatol. (1985) 1 p. 170-175) et F. Barnett et Coll. (Endod. Dent. Traumatol. (1986) 2 p. 71-74) ont montré qu'une pâte aqueuse d'hydroxyde de calcium introduite dans l'endodonte avant fermeture de la cavité par un ciment, avait pendant une durée de plusieurs semaines une activité antibactérienne soutenue, alors que la plupart des antiseptiques utilisés en dentisterie n'étaient efficaces que peu de temps comme l'a montré L. Tronstad et Coll. dans Endod. Dent. Traumatol, 1985, I p. 130-134.

Cette pâte peut être préparée par le praticien qui mélange l'hydroxyde de calcium avec de l'eau distillée, du sérum physiologique ou une solution d 'anesthésique local, ou se trouve dans le commerce sous la forme d'une pâte prête à l'emploi, qui contient Ca(OH)₂ et une solution aqueuse de méthylcellulose ou du soluté de Ringer, et qui est généralement présentée en dose dans une seringue. Mais dans les deux cas, il est difficile d'obtenir une consistance ni trop liquide ni épaisse ou friable, convenant à l'introduction du mélange dans l'endodonte, que ce soit à l'aide de bourre-pâte (Lentulo) ou de fouloirs à canaux.

On a maintenant trouvé une composition qui ne présente pas ces inconvénients.

La composition de désinfection intracanalaire de l'invention est constituée d'un mélange d'hydroxyde de calcium et d'un polyéthylène glycol fluide, de masse moléculaire comprise entre 200 et 600 environ, à raison d'au moins 50 en poids d'hydroxyde de calcium. La composition désinfectante peut contenir, en outre, un colorant, un radioopacifiant, un conservateur et éventuellement un antiinflammatoire, un antiseptique ou un antibiotique pour compléter l'action de Ca(OH)₂. Une composition préférée est constituée d'environ 1/3 de PEG 400 et 2/3 de Ca(OH)₂ en poids.

La composition selon l'invention est préparée par simple mélange des constituants elle peut être présentée sous forme d'unités de dose de 500 mg environ, en plaquettes perforables recouvertes d'un blister ou d'un des petits récipients de quelques millilitres, fermés de préférence de façon étanche.

La composition de l'invention présente de nombreux avantages par rapport aux pâtes aqueuses précédemment connues, et aux mélanges préparés avec d'autres excipients classiquement utilisés en dentisterie, comme l'alcool, le glycérol, qui donnent des compositions trop fluides ou collantes.

Cette composition, biocompatible et non toxique, est hydrophile et Ca(OH)₂ se dissout lentement dans l'eau qui est attirée dans les canaux où la composition a été placée, assurant la protection y compris dans les canaux latéraux ; cette action se prolonge néanmoins plusieurs jours, car la dissolution est lente et une quantité non négligeable de produit actif est introduite grâce à la concentration élevée de Ca(OH)₂ dans la composition, bien que le volume disponible dans les canaux soit faible.

Enfin, elle est facile à éliminer totalement après réouverture de la cavité et le retrait mécanique de la majeure partie, par un simple rinçage avec de l'eau ou une solution aqueuse habituellement utilisée en dentisterie.

La composition est stable et il n'est pas nécessaire de la préparer extemporanément ; il est néanmoins évident que comme tout matériau contenant Ca(OH)₂, elle ne doit pas être abandonnée de façon prolongée à l'air libre pour éviter une prise importante d'humidité et la carbonatation.

Dans ce qui suit, on décrit un exemple de traitement intracanalaire ex vivo avec une pâte selon l'invention.

On a créé une cavité d'accès dans des dents extraites depuis peu de temps et vérifié la vacuité des canaux ; puis on les a nettoyés et mis en forme comme dans un traitement classique de conservation. On a introduit alors environ 25 mg de composition préparée avec 1/3 de polyéthylène glycol 400 et 2/3 de Ca(OH)₂ dans une partie de ces dents pour certaines, la pâte a été placée dans la chambre pulpaire, pour d'autres, elle a été amenée au foramen à l'aide de fouloirs à canaux. La cavité d'accés a été refermée à l'aide d'un ciment provisoire classique et scellée avec un vernis. les dents ont ensuite été immergées dans une solution de bleu Epsilon à 0,5 g/l dans du sérum physiologique. On a constaté qu'au bout de 1 heure, les extrémités canalaires, y compris celles de canaux latéraux, commencent à devenir violettes et le restent plus d'un mois, ce qui traduit l'existence dans cette zone d'un pH élevé et donc la présence prolongée de Ca(OH)₂, tandis que les dents non traitées n'évoluaient pas.

## Revendications

1. Composition pour la désinfection prolongée des canaux dentaires à base d'hydroxyde de calcium, caractérisée en ce qu'elle contient moins de 50 % en poids de polyéthylène glycol de masse moléculaire comprise entre 200 et 600.

2. Composition selon la revendication 1, caractérisée en ce qu'elle comprends en poids de CA(HO)₂ pour 1/3 de polyéthylène glycol 400.

## Claims

1. Composition for the prolonged disinfection of dental canals based on calcium hydroxide, characterized in that it contains less than 50% by weight of polyethylene glycol of molecular mass included between 200 and 600.

2. Composition according to Claim 1, characterized in that it comprises by weight 2/3 of Ca(OH)₂ for 1/3 of polyethylene glycol 400.

## Patentansprüche

1. Zusammensetzung auf der Basis von Calciumhydroxid zur Langzeitdesinfektion von Dentalkanälen, **dadurch gekennzeichnet,** daß sie weniger als 50 Gew.% Polyethylenglycol mit einer Molmasse zwischen 200 und 600 enthält.

2. Zusammensetzung nach Anspruch 1, **dadurch gekennzeichnet,** daß gewichtsmäßig 2/3 Ca(OH)₂ auf 1/3 Polyethylenglycol 400 enthält.
